(19) Europäisches Patentamt
European Patent Office
Office européen des brevets

(11) Veröffentlichungsnummer : **0 047 882**
**B1**

(12) # EUROPÄISCHE PATENTSCHRIFT

(45) Veröffentlichungstag der Patentschrift : **19.12.84**

(51) Int. Cl.³ : **B 01 F 17/52**, D 06 P 3/52

(21) Anmeldenummer : **81106530.9**

(22) Anmeldetag : **22.08.81**

(54) **Formaldehydkondensate enthaltende Dispergiermittel.**

(30) Priorität : **04.09.80 DE 3033329**

(43) Veröffentlichungstag der Anmeldung : **24.03.82 Patentblatt 82/12**

(45) Bekanntmachung des Hinweises auf die Patenterteilung : **19.12.84 Patentblatt 84/51**

(84) Benannte Vertragsstaaten : **CH DE FR GB LI**

(56) Entgegenhaltungen :
**CH-A- 246 989**
**CH-A- 252 305**
**DE-A- 2 934 980**
**DE-B- 1 719 417**
**DE-C- 701 075**

**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Reitz, Gunther, Dr.**
**Semmelweisstrasse 67**
**D-5000 Köln 80 (DE)**
Erfinder : **Jakobs, Karlhans**
**An der Jüch 55**
**D-5060 Bergisch Gladbach 2 (DE)**
Erfinder : **Boehmke, Günther, Dr.**
**Kurt-Schumacher-Ring 152**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind Dispergiermittel, die

1) Anlagerungsprodukte von aliphatischen, araliphatischen oder alkylaromatischen Alkoholen, Carbonsäuren oder Carbonsäureamiden mit 12 bis 22 C-Atomen mit 3-8 Mol Ethylenoxid,

2) Anlagerungsprodukte von aliphatischen, araliphatischen oder alkylaromatischen Alkoholen, Carbonsäuren oder Carbonsäureamiden mit 12 bis 22 C-Atomen mit 9 oder mehr Mol Ethylenoxid und

3) Reaktionsprodukte hergestellt durch Sulfonierung oder Sulfomethylierung und Formaldehydkondensation von Verbindungen der Formel

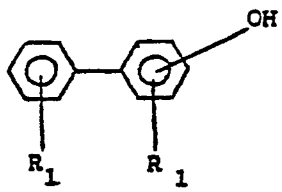

(I)

worin

$R_1$ für $C_{1-4}$-Alkyl steht, und gegebenenfalls anschließende Überführung der entstehenden Sulfonsäuren in ihre Salze, enthalten, und ihre Verwendung beim Färben von Synthesefasern, insbesondere von Polyesterfasern.

Von den Komponenten 1) seien beispielsweise ethoxylierte Fettalkohole, Fettsäuren und Fettsäureamide mit 12-20 C-Atomen sowie Alkylphenole mit 4-16 C-Atomen in der Alkylgruppe genannt. Bevorzugt sind gesättigte und ungesättigte Fettalkohole mit 14-18 C-Atomen, die mit 4-7 Mol Ethylenoxid pro Mol Ausgangsverbindung umgesetzt sind.

Die Komponenten 2) sind beispielsweise ethoxylierte Fettalkohole, Fettsäuren und Fettsäureamide mit 12-20 C-Atomen sowie Alkylphenole mit 4-16 C-Atomen in der Alkylgruppe. Bevorzugt sind Alkylphenole mit 6-12 C-Atomen in der Alkylgruppe, die mit 9-20 Mol Ethylenoxid pro Mol Ausgangsverbindung umgesetzt sind.

In den Komponenten 1) und 2) können die aliphatischen Gruppen gesättigt oder ungesättigt sein.

Von den Komponenten 3) sind diejenigen Kondensate bevorzugt, die durch Sulfonierung und Formaldehydkondensation von (I) entstehen, wobei unter Sulfonierung die Umsetzung mit Chlorsulfonsäure, $SO_3$, Oleum oder $H_2SO_4$ verstanden wird. Bevorzugt ist die Umsetzung mit Oleum und besonders bevorzugt die Umsetzung mit Schwefelsäure.

Die Sulfonierung geschieht vor der Formaldehydkondensation. Die Struktur der dabei entstehenden Produkte ist nicht völlig aufgeklärt. Vermutlich entstehen Verbindungen der allgemeinen Formel

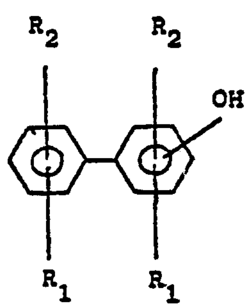

(II)

worin $R_2 = H$, $SO_3H$, mindestens ein $R_2 = SO_3H$ bedeutet.

Es spricht auch einiges dafür, daß noch andere Verbindungen entstehen, z. B. in geringen Mengen das Sulfon von (I) mit der Formel

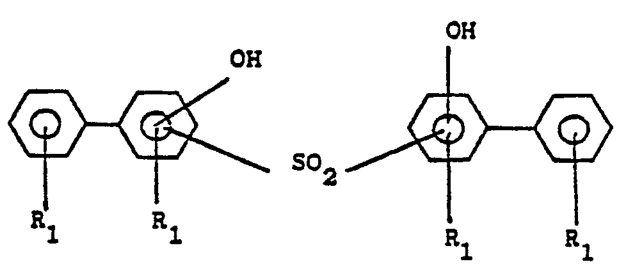

(III)

oder Produkte von bisher nicht aufgeklärter Struktur.

Bei der Sulfomethylierung entstehen Verbindungen, bei denen mindestens einer der aromatischen Ringe von (I) durch den Rest

$$—CH_2—SO_3X$$

worin

X H, Metallkation von Na, K, Ca/2, Mg/2, $N(R_3)_4$ und

$R_3$ H, $C_{1-4}$-Alkyl, das eventuell durch OH substituiert ist,

bedeuten, substituiert ist.

Bei der Formaldehydkondensation der sulfonierten oder sulfomethylierten Verbindungen (I) entstehen hochmolekulare Verbindungen, in denen 2 bis 100, bevorzugt 2-20 der von den Verbindungen (I) abgeleiteten Einheiten enthalten sind. Diese Einheiten sind vor allem durch vom Formaldehyd stammende Methylenbrücken miteinander verknüpft.

Einer oder beide der aromatischen Ringe von (I) können Methylenbrücken tragen. Es ist ebenfalls denkbar, daß an einzelnen aromatische Ringe, besonders wenn sie endständig sind, $—CH_2OH$-Gruppen, die ebenfalls vom Formaldehyd stammen, gebunden sind.

Vor oder bei der Formaldehydkondensation kann ein wasserlöslicher Emulgator zugegeben werden.

Dieser Emulgator kann anionisch, kationisch oder nichtionisch sein. Bevorzugt ist er anionisch oder nichtionisch und besonders bevorzugt nichtionisch. Bei den anionischen Emulgatoren handelt es sich beispielsweise um Alkylsulfonate oder Alkylbenzolsulfonate mit 10 bis 25 C-Atomen im Alkylrest. Die nichtionischen Emulgatoren sind z. B. Umsetzungsprodukte von Fettsäuren, Fettsäureamiden, aliphatischen Alkoholen oder Aminen mit jeweils 10 bis 22 C-Atomen, araliphatischen Alkoholen oder Alkylphenolen mit jeweils 1 bis 20 C-Atomen in der aliphatischen oder Alkyl-Gruppe, die mit 8 bis 50 Äquivalenten Ethylenoxid umgesetzt worden sind. Von den nichtionischen Emulgatoren sind die ethoxylierten Alkohole und die ethoxylierten Alkylphenole besonders bevorzugt.

Im einzelnen sieht das Verfahren zur Herstellung der erfindungsgemäßen Produkte folgendermaßen aus:

Man setzt die Verbindungen (I) mit einem Sulfonierungsmittel, z. B. Chlorsulfonsäure, $SO_3$, Oleum oder besonders bevorzugt Schwefelsäure, im Molverhältnis (I) : Sulfonierungsmittel = 1 : (0,9-2,5) bevorzugt 1 : (1,4-2) um. Bei der Reaktion mit $H_2SO_4$ wird bei 80-180 °C, bevorzugt 110-160 °C in 0,5 bis 10 h, bevorzugt 1-4 h umgesetzt, wobei das Reaktionswasser abdestilliert. Dabei legt man vorteilhaft ein Vakuum von unter 200 Torr, bevorzugt unter 60 Torr an. Der Sulfonierungsgrad, d. h. die Anzahl der an ein Mol der Verbindungen (I) gebundenen $SO_3H$-Gruppen, beträgt 0,8-2,1, bevorzugt 1,0-2,0 Mol.

Nach der Sulfonierung werden in beliebiger Reihenfolge 0,25-15 %, bevorzugt 1-5 % an Emulgator, bezogen auf das Gewicht von (I) und 0,4-4 Mol, pro Mol (I), wäßrige Formaldehydlösung von bevorzugt 20-40 % Gehalt und gegebenenfalls Wasser zugesetzt.

Nach einem bevorzugten Verfahren vermischt man den Emulgator mit der Sulfonsäureschmelze, gibt dann gegebenenfalls Wasser und anschließend die Formaldehydlösung zu. Dabei wird mit 0,4-4 Mol Formaldehyd, bevorzugt 0,6-2 Mol Formaldehyd, pro Mol (I), bei 100-180 °C, bevorzugt 120-160 °C, in 1-10 h, bevorzugt 3-8 h, umgesetzt. Die Formaldehydkondensation kann bei saurem oder alkalischem pH-Wert durchgeführt werden. Bevorzugt ist ein pH von 0-7.

Die sulfomethylierten Verbindungen stellt man bevorzugt im alkalischen Medium aus der Verbindung (I), einem Sulfit, z. B. $Na_2SO_3$, $Na_2S_2O_5$ oder $NaHSO_3$, und Formaldehyd im Molverhältnis 1 : (0,9-2) : (2,4-3) bei 100-180 °C, bevorzugt 120-160 °C in 2-10, bevorzugt 4-8 h dar. Die Darstellung der sulfomethylierten Verbindungen kann auch stufenweise geschehen, indem man zunächst die Verbindung (I) mit — alles berechnet pro Mol (I) — 0,8-2,2 Mol Formaldehyd bei 60-120 °C in 5-120 Minuten, bevorzugt 1,4-2,0 Mol Formaldehyd bei 80-100 °C in 10-60 Minuten, zu den entsprechenden Methylolverbindungen vorkondensiert, die Methylolverbindungen dann mit 0,9-2,2, bevorzugt 1,4-1,9 Mol, Bisulfit bei 80-180 °C in 10-120 Minuten, bevorzugt 100-140 °C in 30-90 Minuten, in die Sulfomethylverbindungen überführt und anschließend mit 0,9-2,8 Mol, bevorzugt 1,0-1,5 Mol, Formaldehyd in 2-10 h bei 100-180 °C, bevorzugt 4-10 h bei 120-160 °C in die Komponenten 3) überführt.

Die Zugabe des Emulgators erfolgt bei der einstufigen Synthese zu Anfang mit den anderen Komponenten. Bei der mehrstufen Synthese wird der Emulgator bevorzugt nach der Herstellung der Sulfomethylverbindungen und vor der Formaldehydkondensation zugegeben.

Es entstehen bräunlich gefärbte 20-40 %ige Lösungen, die zu spröden Pulvern eingetrocknet werden können, die wieder leicht in Wasser löslich sind.

Trotz ihres salzartigen Charakters sind die Komponenten 3) in organischen Lösungsmitteln wie Glykol oder Di-, Tri- und höheren Glykolen völlig homogen löslich.

Die Komponenten 1), 2) und 3) liegen z. B. im Mischungsverhältnis 1 : (0,1-10) : (0,1-40), bevorzugt 1 : (0,5-2) : (0,2-1) vor. Die Summe der Komponenten 1) und 2) steht im Verhältnis zur Komponente 3) von 1 : (0,05-10), bevorzugt 1 : (0,1-1).

In einer konzentrierten wäßrigen Lösung sind die drei Komponenten homogen mischbar. Diese Lösung hat erfahrungsgemäß einen Feststoffgehalt von bis zu 80 %, bevorzugt bis zu 70 %.

Die Vermischung der Komponenten 1), 2) und 3) erfolgt durch Zusammengeben der reinen Komponenten oder ihrer wäßrigen Mischungen bzw. Lösungen. Die Komponente 3) wird vorzugsweise in

wäßriger Lösung eingesetzt, die Komponenten 1) und 2) jedoch vorzugsweise in wasserfreier Form. Nach dem Zusammengeben wird bei Temperaturen bis zu 100 °C unter Rühren vermischt, bevorzugt bei Raumtemperatur. Falls die Mischung nicht klar ist, gibt man Lösungsmittel zu, wobei sie völlig homogen wird. Als Lösungsmittel eignen sich anorganische und organische Verbindungen. Als organische Verbindungen seien solche mit einem hohen Gewichtsverhältnis von Hydroxy-, Amino- und/oder Ethergruppen zum gesamten Molekulargewicht genannt, also Methanol, Ethanol, Glykol, Di-, Tri-, Tetrethylenglykol, Polyglykol, die Mono- und Dimethylether der verschiedenen Glykole, Glycerin, Pentaerythrit, Trimethylolpropan, Mono-, Di-, Tri-ethanolamin sowie Propylenglykol und Butylenglykol. Als Amine seien beispielsweise genannt 1.2- und 1.3-Diamine sowie sich davon ableitende Polyamine.

Geeignet sind ebenfalls die durch Veretherung, Ethoxylierung und/oder Propoxylierung der genannten Verbindungen entstehenden Verbindungen. Von den organischen Verbindungen ist Glykol bevorzugt und von den anorganischen Verbindungen ist Wasser bevorzugt. Besonders bevorzugt ist die Verwendung von Wasser als Lösungsmittel.

Die erfindungsgemäße Mischung der Komponenten 1), 2) und 3) ist ein hervorragendes Egalisiermittel für die Färbung von Synthesefasern, insbesondere von Polyestersynthesefasern, mit organischen Dispersionsfarbstoffen. Die Färbung erfolgt bevorzugt unter Hochtemperaturbedingungen, d. h., in geschlossenen Färbeapparaten bei Temperaturen über 100 °C.

Das erfindungsgemäße Mittel wird der Färbeflotte in Mengen von 0,5-4 g/l, bevorzugt 1-2 g/l, zugesetzt, wobei die g-Menge auf dem im Hilfsmittel enthaltenen Feststoff bezogen ist.

Aus der DE-B-1 719 417 sind Dispergiermittel auf der Grundlage von sulfogruppenhaltigen Phenol-Formaldehyd-Kondensaten bekannt, die in Wasser schwer lösliche Polyalkylenglykolether mehrwertiger Alkohole enthalten.

### Beispiel 1

100 g aus Chlortoluol hergestelltes Oxyditolyl-Isomerengemisch (Verbindung (I), $R_1 = CH_3$) und 80 g Schwefelsäure werden unter Wasserstrahlvakuum drei Stunden auf 150 °C erhitzt und dabei etwa 15 g $H_2O$ abdestilliert. Es werden 2 g eines mit 10 Mol Ethylenoxid umgesetzten Nonylphenols zugegeben und mit der Reaktionsmasse homogen vermischt.

Dann werden 300 ml $H_2O$ zugetropft und unter Verwendung eines pH-Meters mit 45 %iger Natronlauge versetzt, bis ein pH-Wert von 6 erreicht ist. Anschließend werden 50 g einer 30 %igen wäßrigen Formaldehydlösung zugegeben und es wird 6 Stunden auf 145 °C erhitzt.

$\lambda_{max} = 207$ nm (in Wasser)

### Beispiel 2

100 g Oxyditolyl-Isomerengemisch (Verbindung (I), $R_1 = CH_3$) und 100 g Schwefelsäure werden unter Wasserstrahlvakuum drei Stunden auf 140 °C erhitzt und dabei ca. 18 g $H_2O$ abdestilliert. Es werden 3 g eines mit 20 Mol Ethylenoxid umgesetzten Oleylalkohols zugegeben und mit der Reaktionsmasse homogen vermischt. Dann werden 300 ml $H_2O$ zugetropft, und es wird unter Verwendung eines pH-Meters mit 45 %iger Natronlauge bis zu einem pH-Wert von 6 neutralisiert. Anschließend werden 80 g einer 30 %igen wäßrigen Formaldehydlösung zugegeben, und es wird 6 Stunden auf 145 °C erhitzt.

### Beispiel 3

Eine 500 g schwere Kreuzspule wird im Flottenverhältnis 1 : 15 in eine Lösung getaucht, die pro Liter 0,48 g 6fach ethoxylierte Ölsäure, 0,48 g 10fach ethoxyliertes Nonylphenol und 0,75 g der gemäß Beispiel 1 hergestellten Produktlösung enthält. Die wechselseitig zirkulierende Flotte wird auf 75 °C erwärmt und nach Zusatz von 2 g/l Monoatriumsphosphat mit Essigsäure auf pH 4,5-5 eingestellt. Danach werden folgende Farbstoffe in dispergierter Form zugesetzt. (Gewichtsangaben sind bezogen auf Reinfarbstoff) : 0,3 g Disperse Yellow 64 (C.I.-Nr. 47023), 0,25 g Disperse Red 65 (C.I.-Nr. 11228) und 0,36 g Disperse Blue 56 (C.I.-Nr. 63285). Man erwärmt innerhalb von 45-60 Minuten auf 130 °C und färbt 60 Minuten bei dieser Temperatur. Danach wird abgekühlt, gespült und getrocknet.

Die Kreuzspule ist in einem neutralen Grauton gefärbt und zeichnet sich durch eine ausgezeichnete Innen-Außen-Egalität aus.

### Beispiel 4

Es wird wie in Beispiel 3 verfahren, nur enthält die Färbeflotte nicht 0,75 g der gemäß Beispiel 1 hergestellten Lösung, sondern 0,75 g der gemäß Beispiel 2 hergestellten Produktlösung pro Liter.

Die gefärbte Kreuzspule zeichnet sich auch hier durch eine ausgezeichnete Innen-Außen-Egalität aus.

### Beispiel 5

Produkt A = Ölsäure mit 6 Mol Ethylenoxid umgesetzt

Produkt B = Iso-Nonylphenol mit 10 Mol Ethylenoxid umgesetzt
Produkt $C_1$ = Formaldehydkondensat gemäß Beispiel 1
Produkt $C_2$ = Formaldehydkondensat gemäß Beispiel 2.

Die Wirksamkeit der Egalisiermittel für die Färbung von Polyesterfasern mit Dispersionsfarbstoffen wird auch durch die sogenannte Überkochprobe ermittelt, die wie folgt durchgeführt wird :

a) Vorfärbung des Prüfmaterials

Eine Wirkware aus texturiertem Polyester-Filament wird im Flottenverhältnis 1 : 15 mit einer Kombination bestehend aus 0,3 % Disperse Blue 56 (C.I.-Nr. 63285) und 0,1 % Disperse Yellow 5 (C.I.-Nr 12790) berechnet als Reinfarbstoff auf das Warengewicht, ohne spezielles Egalisiermittel, nur unter Zusatz von 1 g/l Mononatriumphosphat und Essigsäure bei pH 4,5 während 1 Stunde bei 130 °C gefärbt. Die gefärbte Ware wird gespült und anschließend mit Natronlauge und Hydrosulfit nachbehandelt.

b) Überkochprobe

Auf einen speziell perforierten, zylindrischen Materialträger werden gleiche Gewichtsteile, und zwar zuerst der gewaschenen, ungefärbten Wirkware aus texturiertem Polyester-Filament und darüber der gemäß a) gefärbten Ware gewickelt und mit Bindfäden befestigt. Die so vorbereitete Probe wird im Flottenverhältnis 1 : 15 mit 2 g/l einer Egalisiermittelmischung, bestehend aus 24 Gewichtsteilen Produkt A, 24 Gewichtsteilen Produkt B, 12 Gewichtsteilen Produkt $C_1$ und 40 Gewichtsteilen $H_2O$, versetzt, mit Mononatriumphosphat/Essigsäure auf pH 4,5 eingestellt und 1 Stunde bei 130 °C mit pulsierender Flotte unter konstanten Pumpbedingungen behandelt. Eine Prüfung der beiden aufgewickelten Wirkwarenstücke zeigt bei beiden eine völlig egale Färbung.

Es wird wie unter 5) verfahren und dabei unter b) 3 g/l einer Egalisiermittelmischung, bestehend aus 16 Gewichtsteilen Produkt A, 16 Gewichtsteilen Produkt B, 8 Gewichtsteilen Produkt $C_2$ und 60 Gewichtsteilen $H_2O$ eingesetzt.

Eine Prüfung der beiden aufgewickelten Wirkwarenstücke zeigt auch hier eine völlig egale Färbung.

## Ansprüche

1. Dispergiermittel enthaltend

1) Anlagerungsprodukte von aliphatischen, araliphatischen oder alkylaromatischen Alkoholen, Carbonsäuren oder Carbonsäureamiden mit 12 bis 22 C-Atomen mit 3-8 Mol Ethylenoxid.

2) Anlagerungsprodukte von aliphatischen, araliphatischen oder alkylaromatischen Alkoholen, Carbonsäuren oder Carbonsäureamiden mit 12 bis 22 C-Atomen mit 9 oder mehr Mol Ethylenoxid und

3) Reaktionsprodukte hergestellt durch Sulfonierung oder Sulfomethylierung und Formaldehydkondensation von Verbindungen der Formel

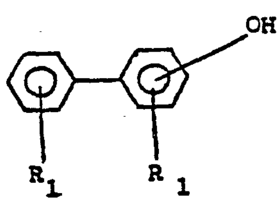

worin

$R_1$ für $C_{1-4}$-Alkyl steht, und gegebenenfalls anschließende Überführung der entstehenden Sulfonsäuren in ihre Salze.

2. Dispergiermittel nach Anspruch 1, enthaltend als Komponente 3) Reaktionsprodukte, hergestellt durch Sulfonierung mit Schwefelsäure und Formaldehydkondensation von Verbindungen der Formel des Anspruchs 1, worin $R_1$ für Methyl steht, und anschließende Überführung in die Natrium-, Kalium-, Calcium-, Magnesium- oder Ammonium-Salze.

3. Dispergiermittel nach Anspruch 1, enthaltend als Komponente 3) Reaktionsprodukte mit 0,8-2,1 Mol $SO_3H$-Gruppen pro Mol der Verbindung der Formel des Anspruchs 1.

4. Dispergiermittel nach Anspruch 1, enthaltend als Komponente 3) Reaktionsprodukte mit 2 bis 100 der von den Verbindungen der Formel des Anspruchs 1 abgeleiteten Einheiten.

5. Dispergiermittel nach Anspruch 1, enthaltend als Komponente 3) Reaktionsprodukte, hergestellt aus der Verbindung der Formel des Anspruchs 1, dem Sulfonierungsmittel und Formaldehyd im Molverhältnis 1 : (0,9-2,5) : (0,4-4).

6. Verwendung der Dispergiermittel des Anspruchs 1 beim Färben von Polyesterfasern.

## Claims

1. Dispersing agents containing

1) addition products of aliphatic, araliphatic or alkylaromatic alcohols, carboxylic acids or carboxylic acid amides with 22 C atoms and 3-8 mols of ethylene oxide,

2) addition products of aliphatic, araliphatic or alkylaromatic alcohols, carboxylic acids or carboxylic acid amides with 12 to 22 C atoms and 9 or more mols of ethylene oxide and

3) reaction products prepared by sulphonation or sulphomethylation and formaldehyde condensation of compounds of the formula

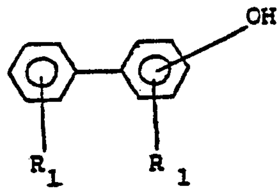

wherein

$R_1$ represents $C_{1-4}$-alkyl, and, if appropriate, subsequent conversion of the resulting sulphonic acids into their salts.

2. Dispersing agents according to Claim 1, containing as component 3) reaction products prepared by sulphonation with sulphuric acid and formaldehyde condensation of compounds of the formula of Claim 1, wherein

$R_1$ represents methyl, and subsequent conversion of the products into the sodium, potassium, calcium, magnesium or ammonium salts.

3. Dispersing agents according to Claim 1, containing as component 3) reaction products containing 0.8-2.1 mols of $SO_3H$ groups per mol of the compound of the formula of Claim 1.

4. Dispersing agents according to Claim 1, containing as component 3) reaction products containing 2 to 100 of the units derived from the compounds of the formula of Claim 1.

5. Dispersing agents according to Claim 1, containing as component 3) reaction products prepared from the compound of the formula of Claim 1, the sulphonating agent and formaldehyde in a molar ratio of 1 : (0.9-2.5) : (0.4-4).

6. Use of the dispersing agents according to Claim 1 in the dyeing of polyester fibres.

**Revendications**

1. Agents dispersants contenant

1) des produits d'addition d'alcools, d'acides carboxyliques ou d'amides d'acides carboxyliques aliphatiques, araliphatiques ou alkylaromatiques ayant 12 à 22 atomes de carbone, avec 3 à 8 moles d'oxyde d'éthylène,

2) des produits d'addition d'alcools, d'acides carboxyliques ou d'amides d'acides carboxyliques aliphatiques, araliphatiques ou alkylaromatiques de 12 à 22 atomes de carbone avec 9 ou plus de 9 moles d'oxyde d'éthylène et

3) des produits de réaction préparés par sulfonation ou sulfométhylation et condensation avec le formaldéhyde de composés de formule

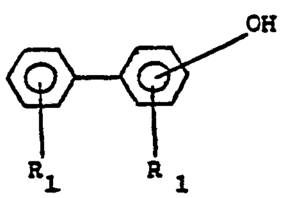

dans laquelle

$R_1$ est un groupe alkyle en $C_1$ à $C_4$, et, le cas échéant, transformation subséquente des acides sulfoniques formés en leurs sels.

2. Agents dispersants suivant la revendication 1, contenant comme composant 3) des produits de réaction préparés par sulfonation à l'acide sulfurique et condensation avec le formaldéhyde de composés de formule suivant la revendication 1, dans laquelle $R_1$ est un groupe méthyle, et transformation subséquente en les sels de sodium, potassium, calcium, magnésium ou ammonium.

3. Agents dispersants suivant la revendication 1, contenant comme composant 3) des produits de réaction portant 0,8-2,1 moles de groupes $SO_3H$ par mole de composé de formule suivant la revendication 1.

4. Agents dispersants suivant la revendication 1, contenant comme composant 3) des produits de réaction avec 2 à 100 des motifs dérivés des composés de formule suivant la revendication 1.

5. Agents dispersants suivant la revendication 1, contenant comme composant 3) des produits de réaction préparés à partir du composé de formule suivant la revendication 1, de l'agent de sulfonation et de formaldéhyde dans la proportion molaire de 1 : (0,9-2,5) : (0,4-4).

6. Utilisation des agents dispersants de la revendication 1 dans la teinture de fibres de polyester.